# EUROPEAN PATENT APPLICATION

(11) **EP 3 778 870 A1**
(43) Date of publication of application: **17.02.2021**
(21) Application number: 19776764.3
(22) Date of filing: 27.03.2019
(51) Int. Cl.: C12N 5/02, C12N 5/071, C12N 5/077

(54) **CELL MASS FUSION METHOD**

(30) Priority: 28.03.2018 JP 2018061095
(71) Applicant: Public University Corporation Yokohama City University, Yokohama-shi, Kanagawa 236-0027 (JP)
(72) Inventor: TANIGUCHI, Hideki, Yokohama-shi, Kanagawa 236-0004 (JP); TADOKORO, Tomomi, Yokohama-shi, Kanagawa 236-0004 (JP)
(74) Representative: J A Kemp LLP
(86) International application number: PCT/JP2019/013114
(87) International publication number: WO 2019/189324

(57) **Abstract**

The present invention provides a method of fusing cell masses. A method of fusing cell masses, comprising seeding cell masses on a plane capable of cell adhesion and culturing the cell masses as a culture medium is fed from both the obverse and reverse sides of the cell mass-seeded plane.

## Description

### TECHNICAL FIELD

The present invention relates to a method of fusing cell masses.

### BACKGROUND ART

Vascular systems exist in almost all tissues/organs in a living body and are playing the roles of supplying blood cells, oxygen, nutrients, etc. and removing waste products, etc.

Recently, three-dimensional culture methods by *in vitro* cell culture using various epithelial cells (of intestine, liver, or the like) have been established (Non-Patent Documents Nos. 1 and 2). However, the products obtained by such methods are organoids consisting of epithelial cells alone, and vascular structures are not contained therein.

Following such techniques, new techniques for preparing organoids (organ primordia) provided with microvascular networks by mixing progenitor cells of an organ of interest, vascular endothelial cells and mesenchymal cells and initiating autonomous cell aggregation on a matrix such as Matrigel™ have been developed (Non-Patent Document No. 3 and Patent Document No. 1).

Use of a low attachment plate or the like makes it possible to prepare cell masses without using a matrix. However, mainly because of the problem of demand for nutrients and oxygen, the size of cell masses should not exceed several hundred micrometers in order to realize prolonged culture without causing internal necrosis (Non-Patent Document No. 4).

### PRIOR ART LITERATURE

### Non-Patent Documents

Non-Patent Document No. 1: Nature 459, 262-265, 2009
Non-Patent Document No. 2: Cell 160, 299-312, 2015
Non-Patent Document No. 3: Nature 399, 481-484, 2013
Non-Patent Document No. 4: Br. J. Cancer (1986), 53, 345-353

### Patent Document

Patent Document No. 1: WO2013/047639

### DISCLOSURE OF THE INVENTION

### PROBLEM FOR SOLUTION BY THE INVENTION

Although it is essential to use a matrix for preparing organoids with diameters of several hundred micrometers and more, the matrix around organoids inhibits their aggregation/fusion, making it difficult to produce large enough organoids in sizes of 5 mm and more. Further, from the viewpoint of conformity to GCTP, the use of a matrix is a factor that makes it difficult to apply organoids to regenerative medicine and other fields.

It is an object of the present invention to provide a method of preparing large size organoids without using a matrix.

### MEANS TO SOLVE THE PROBLEM

The present inventors prepared organoids (cell masses) by co-culturing organ cells, vascular endothelial cells and mesenchymal cells in a matrix-free culture vessel, seeded these organoids on a plane capable of cell adhesion, and cultured the organoids as a culture medium was fed from both the obverse and reverse sides of the organoid-seeded plane, whereby fused organoids of any size could successfully be generated, leading to a completion of the present invention. The present inventors have then confirmed that the fused organoids form a vascular network structure in the inside. It was also observed with the fused organoids that they had elevated levels of gene expression of two or more organ differentiation markers. In fused liver buds prepared by the method of the present invention, liver function related genes were found to be increased in expression level, and formation of microvessels or arterioles/venules, sinusoids, and intrahepatic bile ducts was also confirmed. Furthermore, when the fused organoid was transplanted into mice, anastomosis and blood perfusion between the mouse vascular system and the vascular system of the fused organoid were confirmed.

A summary of the present invention is as described below.
(1) A method of fusing cell masses, comprising seeding cell masses on a plane capable of cell adhesion and culturing the cell masses as a culture medium is fed from both the obverse and reverse sides of the cell mass-seeded plane.
(2) The method of (1) above, wherein cell masses are seeded on the plane capable of cell adhesion in such a manner that the ratio of the area occupied by the cell masses to the seeded plane is 40 to 100%.
(3) The method of (1) or (2) above, wherein the cell mass comprises vascular cells and/or mesenchymal cells.
(4) The method of (3) above, wherein the ratio of mesenchymal cells to vascular cells is 1 : 0.01-100.
(5) The method of (4) above, wherein the ratio of mesenchymal cells to vascular cells is 1 : 0.1-10.
(6) The method of any one of (1) to (5), wherein the resultant fused cell mass is forming a vascular network structure.
(7) The method of any one of (1) to (6), wherein the cell mass is an organ bud.
(8) The method of (7) above, wherein the organ bud has been formed from tissue or organ cells, mesenchymal cells and vascular cells.
(9) The method of (8) above, wherein the ratio of tissue or organ cells, mesenchymal cells and vascular cells is 10 : 0.1-10 : 0.1-10.
(10) The method of (9) above, wherein the ratio of mesenchymal cells to vascular cells is 1 : 0.1-10.
(11) The method of any one of (7) to (10), wherein the organ bud has been formed in a culture vessel having a non-cell-adhesive plane.
(12) The method of any one of (7) to (11), wherein the organ bud is a liver bud and the fused liver bud is forming a vascular network structure.
(13) The method of (12) above, wherein the vascular network structure comprises microvessels and/or arterioles/venules.
(14) The method of any one of (6) to (13) above, wherein the organ bud is a liver bud and the fused liver bud is forming a bile duct structure.
(15) A method of preparing a fused cell mass, comprising seeding cell masses on a plane capable of cell adhesion and culturing the cell masses as a culture medium is fed from both the obverse and reverse sides of the cell mass-seeded plane to thereby fuse the cell masses.
(16) A method of constructing a vascular network structure, comprising seeding cell masses on a plane capable of cell adhesion and culturing the cell masses as a culture medium is fed from both the obverse and reverse sides of the cell mass-seeded plane to thereby form a vascular network structure.
(17) An artificial organ that is prepared by fusing liver buds *in vitro* as they have been formed from hepatocytes, mesenchymal cells and vascular cells and which has a vascular network structure and/or a bile duct structure.
(18) The artificial organ of (17) above, wherein at least one of the hepatocyte, the mesenchymal cell and the vascular cell has been generated by directed differentiation from induced pluripotent stem cells.

According to the present invention, it is possible to prepare a fused cell mass of millimeter order or larger. According to the present invention, it is also possible to form a vascular network-like structure in a fused cell mass with good reproducibility. Furthermore, according to the present invention, it is possible to prepare a fused liver bud with elevated expression levels of liver function related genes. In such fused liver buds, microvessels or arterioles/venules, sinusoids, and intrahepatic bile ducts can be formed.

According to the present invention, fused organoids with a vascular network structure can be prepared consistently, so by means of further fusing them with a tissue such as the great vessel, transplantable organs could be prepared in the future.

### EFFECT OF THE INVENTION

According to the present invention, it has become possible to prepare organoids of large size.

The present specification encompasses the contents disclosed in the specification and/or drawings of Japanese Patent Application No. 2018-061095 based on which the present patent application claims priority.

### BRIEF DESCRIPTION OF THE DRAWINGS

[Fig. 1] shows the results of examination of cell mass fusion using small size liver buds.
[Fig. 2] shows the results of examination of cell mass fusion using conventional liver buds (i.e., liver buds prepared on Matrigel™).
[Fig. 3] shows the results of tissue reconstitution in fused liver buds.
[Fig. 4] shows the results of analysis of hepatic differentiation markers in small size liver buds and fused liver buds.
[Fig. 5] shows the results of analysis of hepatic functions in conventional liver buds and fused liver buds.
[Fig. 6] shows the results of transplantation of fused liver buds into cranial window.
[Fig. 7] shows the results of controlling vascular network formation by changing the proportions of three constituent cell types.
[Fig. 8] shows the results of size control in fused liver buds.
[Fig. 9] shows the results of examination of the size of cell masses and the density of seeding.
[Fig. 10] shows the results of examination of the proportions of three cell types.

### BEST MODES FOR CARRYING OUT THE INVENTION

Hereinbelow, the present invention will be described in detail.

The present invention provides a method of fusing cell masses, comprising seeding cell masses on a plane capable of cell adhesion and culturing the cell masses as a culture medium is fed from both the obverse and reverse sides of the cell mass-seeded plane. According to the method of the present invention, a fused cell mass can be obtained. Therefore, the present invention provides a method of preparing a fused cell mass, comprising seeding cell masses on a plane capable of cell adhesion and culturing the cell masses as a culture medium is fed from both the obverse and reverse sides of the cell mass-seeded plane to thereby fuse the cell masses. According to the method of the present invention, it is possible to construct a vascular network structure in cell masses. Therefore, the present invention provides a method of constructing a vascular network structure, comprising seeding cell masses on a plane capable of cell adhesion and culturing the cell masses as a culture medium is fed from both the obverse and reverse sides of the cell mass-seeded plane to thereby form a vascular network structure.

According to the present invention, it is possible to prepare a cell mass of 8 mm or more in diameter, which is a size that has not been reported with conventional methods where cell masses are prepared without using a matrix. According to Br. J. Cancer (1986), 53, 345-353, the plotted data seem to indicate that cell masses up to 2.2 mm in diameter are formed. According to other papers, it seems that cell masses up to 1.1 mm in diameter can be prepared with rotary culture equipment (Zanoni M et al., Sci. Rep. 6, 19103. 2016); that cell masses of 1 mm in diameter can be prepared on U-bottom plates (Vinci M et al., BMC Biology 10:29, 2012); that cell masses of 4 mm in diameter can be obtained by first preparing cell masses on U-bottom plates and then performing gyrotary culture on low adhesion plates (Xiang Y et al., Cell Stem Cell 21, 383-398, 2017); and that cell masses up to 4 mm in diameter can be prepared on a cell culture insert (Takasato M et al., Nature 526, 564-568, 2015). On the other hand, according to methods in which organoids are prepared using a matrix, the maximum obtainable size seems to be about 5 mm (Chen Y et al., Nat. Cell Biol. 19, 542-549). According to the present invention, it is possible to prepare cell masses larger than the cell masses reported in this paper.

The cell mass may be any cell assembly such as organ buds (organoids) or spheroids. Preferably, the cell mass comprises either one or both of vascular cells (e.g., vascular endothelial cells) and mesenchymal cells (e.g., undifferentiated mesenchymal cells such as mesenchymal stem cells). The ratio of mesenchymal cells to vascular cells may be 1 : 0.01-100, preferably 1 : 0.1-10, and more preferably 1 : 0.5-2. When cell masses comprise vascular cells and mesenchymal cells, a vascular network structure may be formed in the resultant fused cell mass.

A spheroid is a spherical cell assembly in which cells are assembled/aggregated to provide a three-dimensional structure.

The term "organ bud" means a structure capable of differentiating into an organ through maturing. As one example of such organ bud, WO2013/047639 discloses a method of preparing an organ bud from three types of cells which are tissue or organ cells, vascular cells (for example, vascular endothelial cells) and mesenchymal cells (for example, undifferentiated mesenchymal cells such as mesenchymal stem cells or cells differentiated therefrom). In the present invention, organ buds prepared by this method may suitably be used. Whether a structure is an organ bud or not can be determined, for example, by transplanting the structure into an organism and examining whether or not it is capable of differentiating into an organ of interest (the structure can be judged as organ bud if it has differentiated into the organ of interest); and/or by examining whether or not the structure comprises all of the above-described three types of cells, i.e., tissue or organ cells, vascular endothelial cells and mesenchymal cells (the structure can be judged as organ bud if it comprises all of the three types of cells). The organ bud may be one which differentiates into an organ such as kidney, heart, lung, spleen, esophagus, stomach, thyroid, parathyroid, thymus, gonad, brain, spinal cord or the like. Preferably, the organ bud is one which differentiates into an endodermal organ such as one which differentiates into liver (liver bud), one which differentiates into pancreas (pancreas bud), or one which differentiates into intestinal tract. Whether a certain structure is an organ bud which differentiates into an endodermal organ or not can be determined by examining the expression of marker proteins (if any one or more of the marker proteins described later are expressed, the organ bud can be judged as the organ bud of interest). For example, HHEX, SOX2, HNF4A, AFP, ALB and the like are markers for liver bud; PDX1, SOX17, SOX9 and the like are markers for pancreas bud; and CDX2, SOX9 and the like are markers for organ buds which differentiate into intestinal tract. Among the terms used by those skilled in the art, the following are included in the organ bud of the present invention: liver bud, liver diverticula, liver organoid, pancreatic (dorsal or ventral) buds, pancreatic diverticula, pancreatic organoid, intestinal bud, intestinal diverticula, intestinal organoid (K. Matsumoto, et al. Science. 19; 294 (5542): 559-63 (2001)) and so on.

In the present invention, the term "tissue or organ cell" means functional cells constituting tissues or organs, or undifferentiated cells which differentiate into functional cells. Examples of "undifferentiated tissue or organ cell" include, but are not limited to, cells capable of differentiating into an organ such as kidney, heart, lung, spleen, esophagus, stomach, thyroid, parathyroid, thymus, gonad, brain or spinal cord; cells capable of differentiating into an ectodermal organ such as brain, spinal cord, adrenal medulla, epidermis, hair/nail/dermal gland, sensory organ, peripheral nerve or lens; cells capable of differentiating into a mesodermal organ such as kidney, urinary duct, heart, blood, gonad, adrenal cortex, muscle, skeleton, dermis, connective tissue or mesothelium; and cells capable of differentiating into an endodermal organ such as liver, pancreas, intestinal tract, lung, thyroid, parathyroid or urinary tract. Whether or not a cell is capable of differentiating into an ectodermal organ, mesodermal organ or endodermal organ can be determined by examining the expression of marker proteins (if any one or more of marker proteins are expressed, the cell can be judged as a cell capable of differentiating into an endodermal organ). For example, in cells capable of differentiating into liver, HHEX, SOX2, HNF4A, AFP, ALB and the like are markers; in cells capable of differentiating into pancreas, PDX1, SOX17, SOX9 and the like are markers; in cells capable of differentiating into intestinal tract, CDX2, SOX9 and the like are markers; in cells capable of differentiating into kidney, SIX2 and SALL1 are markers; in cells capable of differentiating into heart, NKX2-5, MYH6, ACTN2, MYL7 and HPPA are markers; in cells capable of differentiating into blood, C-KIT, SCA1, TER119 and HOXB4 are markers; and in cells capable of differentiating into brain or spinal cord, HNK1, AP2, NESTIN and the like are markers. Among the terms used by those skilled in the art, the following are included in the "undifferentiated tissue or organ cell" of the present invention: hepatoblast, hepatic progenitor cells, hepatic precursor cells, pancreatoblast, pancreatic progenitors, pancreatic progenitor cells, pancreatic precursor cells, endocrine precursors, intestinal progenitor cells, intestinal precursor cells, intermediate mesoderm, metanephric mesenchymal precursor cells, multipotent nephron progenitor, renal progenitor cells, cardiac mesoderm, cardiovascular progenitor cells, cardiac progenitor cells (JR. Spence, et al. Nature.; 470(7332):105-9.(2011); Self, et al. EMBO J.; 25(21): 5214-5228.(2006); J. Zhang, et al. Circulation Research.; 104: e30-e41(2009); G. Lee, et al. Nature Biotechnology 25, 1468-1475 (2007)) and so on. Undifferentiated tissue or organ cells may be collected from tissues or organs, or may be prepared from pluripotent stem cells such as induced pluripotent stem cells (iPS cells) or embryonic stem cells (ES cells) according to known methods. Moreover, undifferentiated tissue or organ cells may be such cells as primitive gut endoderm cells (PGECs) (Japanese Patent No. 5777127) which are at an intermediate stage of differentiation from pluripotent stem cells (e.g., iPS cells) into tissues or organs. PGECs are capable of differentiating into hepatocytes, pancreatic cells and enterocytes (have high differentiation function), do not express markers associated with the malignancy of cancer (are highly safe), and may be prepared from iPS cells by directed differentiation without using feeder cells. Therefore, PGECs have the advantage of even allowing for clinical application. Furthermore, mass preparation of PGECs is possible. PGECs may be prepared according to the method disclosed in Japanese Patent No. 5777127. Alternatively, pluripotent stem cells such as iPS cells may be cultured in activin-supplemented serum-free medium so that they are induced to endodermal cells that are positive to both CXCR4 and E-cadherin; or the endodermal cells thus obtained may be cultured for two days in the presence of added BMP4 and FGF2 to obtain CXCR4-negative, HNF4α-positive hepatic endodermal cell populations. To give further examples, organ cells capable of differentiating into liver may be prepared as previously described (K. Si-Taiyeb, et al. Hepatology, 51 (1): 297- 305(2010); T. Touboul, et al. Hepatology. 51 (5): 1754-65 (2010)); organ cells capable of differentiating into pancreas may be prepared as previously described (D. Zhang, et al. Cell Res.;19(4):429-38 (2009)); organ cells capable of differentiating into intestinal tract may be prepared as previously described (J. Cai, et al. J Mol Cell Biol.; 2(1):50-60 (2010); R. Spence, et al. Nature.; 470 (7332): 105-9 (2011)); cells capable of differentiating into heart may be prepared as previously described (J. Zhang, et al. Circulation Research.; 104: e30-e41(2009); and cells capable of differentiating into brain or spinal cord may be prepared as previously described (G. Lee, et al. Nature Biotechnology 25, 1468 - 1475 (2007)). Examples of "differentiated tissue or organ cell" include, but are not limited to, endocrine cells of pancreas, pancreatic duct epithelial cells of pancreas, hepatocytes of liver, epithelial cells of intestinal tract, tubular epithelial cells of kidney, podocytes of kidney, cardiomyocytes of heart, lymphocytes and granulocytes of blood, erythrocytes, neurons and glial cells of brain, and neurons and Schwann cells of spinal cord. Tissue or organ cells may be cells derived from malignantly transformed tissue or organ (i.e., cancer cells). Cancer cell may be any of established cancer cell lines, primary cultured or subcultured cancer cells derived from cancer patients (including non-human animal). As tissue or organ cells, human-derived cells are mainly used. However, tissue or organ cells derived from non-human animals (e.g., animals used, for example, as experimental animals, pet animals, working animals, race horses or fighting dogs; more specifically, mouse, rat, rabbit, pig, dog, monkey, cattle, horse, sheep, chicken, shark, devilfish, ratfish, salmon, shrimp, crab or the like) may also be used.

Although vascular cells may be isolated from vascular tissues such as umbilical veins, vascular cells are not limited to those isolated from vascular tissues and may be induced from totipotent or pluripotent cells (e.g., iPS cells or ES cells) by directed differentiation. As vascular cell, vascular endothelial cell, vascular smooth muscle cell or the like may be enumerated. Among them, vascular endothelial cell is preferable. Vascular endothelial cells derived from umbilical vein are commercially available and easy to access. In the present invention, the term "vascular endothelial cell" means cells constituting vascular endothelium or cells capable of differentiating into such cells. Whether a cell is vascular endothelial cell or not can be determined by examining the expression of marker proteins such as TIE2, VEGFR-1, VEGFR-2, VEGFR-3 and CD41 (if any one or more of the above-listed marker proteins are expressed, the cell can be judged as vascular endothelial cell). The vascular endothelial cell used in the present invention may be either differentiated or undifferentiated. Whether a vascular endothelial cell is a differentiated cell or not can be determined by means of CD31 and CD144. Among the terms used by those skilled in the art, the following are included in the "vascular endothelial cell" of the present invention: endothelial cells, umbilical vein endothelial cells, endothelial progenitor cells, endothelial precursor cells, vasculogenic progenitors, hemangioblast (HJ. Joo, et al. Blood. 25;118(8):2094-104 (2011)) and so on. Preferable vascular endothelial cells are those derived from umbilical vein. Vascular endothelial cells may be isolated from blood vessels, or prepared from pluripotent stem cells (such as iPS cell, ES cells, etc.) according to known methods. As vascular endothelial cells, human-derived cells are mainly used. However, vascular endothelial cells derived from non-human animals (e.g., animals used, for example, as experimental animals, pet animals, working animals, race horses or fighting dogs; more specifically, mouse, rat, rabbit, pig, dog, monkey, cattle, horse, sheep, chicken, shark, devilfish, ratfish, salmon, shrimp, crab or the like) may also be used.

In the present invention, the term "mesenchymal cell" means connective tissue cells that are mainly located in mesoderm-derived connective tissues and which form support structures for cells that function in tissues. The "mesenchymal cell" is a concept that encompasses those cells which are destined to, but are yet to, differentiate into mesenchymal cells. Mesenchymal cells used in the present invention may be either differentiated or undifferentiated. Whether a certain cell is an undifferentiated mesenchymal cell or not may be determined by examining the expression of marker proteins such as Stro-1, CD29, CD44, CD73, CD90, CD105, CD133, CD271 or Nestin (if any one or more of the above-listed marker proteins are expressed, the cell can be judged as an undifferentiated mesenchymal cell). A mesenchymal cell in which none of the above-listed markers are expressed can be judged as a differentiated mesenchymal cell. Among the terms used by those skilled in the art, the following are included in the "mesenchymal cell" of the present invention: mesenchymal stem cells, mesenchymal progenitor cells, mesenchymal cells (R. Peters, et al. PLoS One. 30; 5(12):e15689 (2010)) and so on. Preferable mesenchymal cells are mesenchymal cells derived from bone marrow (especially, mesenchymal stem cells). Mesenchymal cells may be isolated from tissues such as bone marrow, fat tissue, placental tissue, umbilical tissue, dental pulp, etc. or prepared from pluripotent stem cells (such as iPS cell, ES cells, etc.) according to known methods. As mesenchymal cells, human-derived cells are mainly used. However, undifferentiated mesenchymal cells derived from non-human animals (e.g., animals used, for example, as experimental animals, pet animals, working animals, race horses or fighting dogs; more specifically, mouse, rat, rabbit, pig, dog, monkey, cattle, horse, sheep, chicken, shark, devilfish, ratfish, salmon, shrimp, crab or the like) may also be used.

A method is disclosed in Takebe T et al., 2017, Cell Reports 21, 2661-2670 in which all the three types of cells (i.e., tissue or organ cells, vascular endothelial cells and mesenchymal cells) are prepared from iPS cells, and organ buds are generated from the resultant cells. These cells and organ buds disclosed therein may be used in the present invention.

Culture ratios of the three cell types in coculture are not particularly limited as long as the ratio enables the formation of organ buds. A preferable cell count ratio is as follows. Tissue or organ cell : vascular cell : mesenchymal cell = 10 : 0.1-10 : 0.1-10. With less vascular cells and mesenchymal cells, formation of blood vessels becomes difficult. With more mesenchymal cells, adhesivity among cells becomes stronger than adhesion to the plane capable of cell adhesion, whereby cells often contract, making fusion of cell masses difficult. With more vascular cells, shortage occurs in the amount of mesenchymal cells that are necessary for constructing blood vessels, whereby vascular formation is inhibited. Cell count ratio between mesenchymal cells and vascular cells may be 1 : 0.01-100, preferably 1 : 0.1-10, and more preferably 1 : 0.5-2. It is possible to form an organ bud of approx. 3,500-4,500 micrometers in size by coculturing around 230,000 tissue or organ cells, around 160,000 vascular endothelial cells and around 160,000 mesenchymal sells. As the ratio of mesenchymal cells increases, a vascular network with a finer network structure will be formed in organ buds. A cell count ratio suitable for forming a vascular network structure is tissue or organ cell : vascular endothelial cell : mesenchymal cell = 10 : 1-10 : 1-10, more preferably 10 : 2-7 : 2-7.

The medium used for coculturing cells for organ bud formation is not particularly limited. Any medium may be used as long as it enables the formation of organ buds. Preferably, a medium for culturing endothelial vascular cells, a medium for culturing tissue or organ cells or a mixture of these two media may be used. As a medium for culturing endothelial vascular cells, any medium may be used but, preferably, a medium containing at least one of the following substances may be used: hEGF (recombinant human epidermal growth factor), VEGF (vascular endothelial growth factor), hydrocortisone, bFGF, ascorbic acid, IGF1, FBS, antibiotics (e.g., gentamycin or amphotericin B), heparin, L-glutamine, phenol red and BBE. Specific examples of this medium which may be used in the present invention include, but are not limited to, EGM-2 BulletKit (Lonza), EGM BulletKit (Lonza), VascuLife EnGS Comp Kit (LCT), Human Endothelial-SFM Basal Growth Medium (Invitrogen) and human microvascular endothelial cell growth medium (TOYOBO). As a medium for culturing tissue or organ cells, any medium may be used but, when the organ cell is hepatocyte, a medium containing at least one of the following substances may be preferably used: ascorbic acid, BSA-FAF, insulin, hydrocortisone and GA-1000. As a medium for culturing hepatocyte, HCM BulletKit (Lonza) from which hEGF (recombinant human epidermal growth factor) has been removed and RPMI1640 (Sigma-Aldrich) to which 1% B27 Supplements (GIBCO) and 10 ng/mL hHGF (Sigma-Aldrich) have been added may typically be used. With respect to formation of human liver buds, use of a medium prepared as described below has been found effective for maturation of liver buds. Briefly, EGM BulletKit (Lonza) and HCM BulletKit (Lonza) from each of which hEGF has been removed are mixed at 1:1 and to the resultant mixture, dexamethasone, oncostatin M and HGF are added.

The temperature at the time of culture for organ bud formation is not particularly limited. The temperature is preferably 30-40 °C, more preferably 37 °C.

The time period of culture for organ bud formation is not particularly limited. The period is preferably 3-14 days, more preferably 10 days.

In the present invention, the cell mass (such as organ bud) is preferably prepared in a matrix-free manner, that is, without using a plane capable of cell adhesion such as a matrix. For example, the cell mass may be one which is formed in culture equipment with a non-cell-adhesive plane.

The culture equipment with a non-cell-adhesive plane may be one that has received a low adsorption surface treatment. For example, culture equipment whose culture surface is coated with a non-cell-adhesive polymer may be used. Examples of non-cell-adhesive polymer include, but are not limited to, phospholipids, complexes of phospholipid and polymer, poly(2-hydroxyethylmethacrylate) (PHEMA), polyvinyl alcohol, agarose, chitosan, polyethylene glycol, albumin, and photocrosslinkable super-hydrophilic polymers.

The bottom of the culture equipment may suitably have a large number of concavities in a semi-spherical or truncated conical shape. For example, using culture equipment as a 24-well plate having 600 concavities in a semi-spherical or truncated conical shape (volume of each concavity: 0.068 mm³) per well, cell masses may be formed by culturing a total of 200,000 to 3,000,000 cells per well. Then, the size of cell mass may reach 80-500 micrometers. The above-described culture equipment and culture conditions are disclosed in WO2015/182159 and are cited herein by reference. Examples of culture equipment with a non-cell-adhesive plane include, but are not limited to, Elplasia RB 500 400 NA (Kuraray) and 96-well U bottom plate or V bottom plate (Sumitomo Bakelite). These can be suitably used in the present invention.

In the present invention, cell masses may be seeded on a plane capable of cell adhesion in such a manner that the ratio of the area occupied by cell masses to the seeded plane is 40 to 100%. The ratio of the area occupied by cell masses to the seeded plane is preferably 60 to 100% and more preferably 80 to 100%. The ratio of the area occupied by cell masses to the seeded plane can be obtained by measuring the projected shadow area of cell masses and calculating the ratio of this area to the area of seeded plane. The projected shadow area may be measured by the following method. Briefly, the projected shadow area of cell masses is calculated with image analysis software such as FIJI, ImageJ, Photoshop, etc.

In the method of the present invention, cell masses may be seeded on a plane capable of cell adhesion at high density. For example, assuming a cell mass diameter is 150 µm, high density means that the number of cell masses present per cm³ of space may be 9.5 x 10⁴ to 3.8 x 10⁵, preferably 1.9 x 10⁵ to 3.8 x 10⁵, more preferably 2.9 x 10⁵ to 3.8 x 10⁵.

The number of cell masses may be two or more. When the number of cell masses is increased, fused cell mass of larger size can be obtained (see Examples described later).

The size of cell mass is appropriately 80-500 µm, preferably 100-250 µm.

The culture medium is not particularly limited. Any medium may be used as long as it is suitable for culturing cell masses. For example, if the cell mass is a liver bud, examples of preferable media include, but are not limited to, a medium obtained by adding dexamethasone, oncostatin M and HGF to a 1:1 mixture of EGM BulletKit (Lonza) and HCM BulletKit (Lonza) from which hEGF (recombinant human epithelial cell growth factor) has been removed; a medium which is a 1:1 mixture of EGM BulletKit (Lonza) and VascuLife EnGSD Comp Kit (LCT); and a medium which is a 1:1 mixture of EGM BulletKit (Lonza) and Endothelial Cell Growth Medium MV.

The term "fusion among cell masses" means that a plurality of cell masses form a continuous structure. Cell masses fused are capable of constructing a continuous vascular structure through internal self-organization. Fusion among cell masses may achieve the following: the size of cell mass is increased; a vascular network structure is formed in cell mass; vascular network structure is developed further; and the function of cell mass is improved.

In the present invention, cell masses are fused by seeding cell masses on a plane capable of cell adhesion and culturing the cell masses as a culture medium is fed from both the obverse and reverse sides of the cell mass-seeded plane.

Fusion of cell masses is performed on a plane capable of cell adhesion. If, for example, the plane capable of cell adhesion assumes the structure of a porous membrane, it would be advantageous for culturing cell masses after fusion because nutrients can be fed to fused cell masses from both top and bottom while allowing for efficient oxygen supply. However, the applicable plane is not limited to this embodiment. Examples of planes capable of cell adhesion include, but are not limited to, planes which have been negatively charged to acquire hydrophilicity by means of corona discharge in the atmosphere or vacuum gas plasma polymerization treatment (cell adhesion surface treatment) or the like; planes with a gelatin treated surface; planes coated with extracellular matrix (such as collagen, laminin, or fibronectin) or mucopolysaccharides (heparin sulfate, hyaluronic acid, chondroitin sulfate, etc.); planes coated with basic synthetic polymers (such as poly-D-lysine); planes with a synthetic nanofiber surface; planes with a hydrophilic and neutral hydrogel layer's surface; and collagen membrane (KOKEN CO., LTD.). In the case where the plane capable of cell adhesion assumes the structure of a porous membrane, the pore size may be 0.4-8 µm. Examples of culture equipment with a plane capable of cell adhesion include, but are not limited to, Falcon cell culture plate (Corning), Falcon multi-cell culture plate (Corning), and Falcon cell culture insert (Corning). These may be used advantageously in the present invention. Culture may be performed in any method selected from among batch culture, semi-batch culture (fedbatch culture) and continuous culture (perfusion culture). Culture may be either static culture, aeration culture, spinner culture, shaking culture or rotary culture. Among these, static culture is preferable.

Fusion of cell masses may be performed, for example, on a plane capable of cell adhesion in culture equipment. Alternatively, fusion of cell masses may also be performed on a plane capable of cell adhesion in a heterologous construct. The term "heterologous" in heterologous construct means being derived from a different source than cell masses. The construct derived from a different source than cell masses may be suitably a construct with a biological or a resembling function. As regards such constructs, biological tissues or organs derived from a different organism than cell masses, and artificial tissues and organs may be enumerated. Among these, a construct having a luminal structure is preferable. Specific examples of heterologous constructs include, but are not limited to, biological tissues and organs such as vessel, bile duct, intestinal tract, esophagus, pancreatic duct, trachea, ureter or oviduct; artificial tissues and organs such as artificial blood vessel, artificial trachea or cell sheet; sheet-like constructs; tube-like constructs; and thread-like constructs. The heterologous construct may be formed from a bio-compatible material. As regards bio-compatible materials, metals (such as stainless steel, cobalt alloy, or titanium alloy), glass, ceramics, synthetic polymers (such as nylon, polypropylene, polydioxanone, polylactic acid, polyethylene terephthalate, or Teflon™), biomaterials (such as silk, collagen, or decellularized tissue) or the like may be enumerated, but bio-compatible materials are not limited to these materials. A plane capable of cell adhesion is as defined above.

The temperature at the time of culture for fusing cell masses is not particularly limited. The temperature is preferably 25-37 °C.

The time period of culture for fusing cell masses is not particularly limited. The period is preferably 1-10 days.

According to the present invention, it is possible to prepare large sized cell masses without using a support such as Matrigel™.

According to the method of the present invention, it is possible to prepare fused cell masses with diameters of 100 µm or more, 1 mm or more, 2 mm or more, 2.5 mm or more, 4 mm or more, 6 mm or more, and 8 mm or more. Fused cell masses with diameters of 100 µm or more, 1 mm or more, 2 mm or more, 2.5 mm or more, 4 mm or more, 6 mm or more, and 8 mm or more may be prepared from 2 to 4 cell masses, 150-200 cell masses, 300-400 cell masses, 350-500 cell masses, 600-800 cell masses, 1200-1600 cell masses, and 2400-2800 cell masses, respectively, with a size of about 80-150 µm.

Further, the fused cell mass prepared by the method of the present invention is capable of forming a vascular network structure. In one example described later, a vascular network structure was formed in fused liver bud. As regards the vascular network structure, microvessels, arterioles/venules, sinusoids, or the like may be enumerated. Furthermore, a fused liver bud is capable of forming a bile duct structure.

Cell masses fused by the method of the present invention may have an improved function compared to the function of cell masses before fusion. For example, in the case where the cell mass is a liver bud, the fused liver bud can have higher gene expression levels of hepatic differentiation markers (e.g., FoxA2, AFP, CYP3A7 and CYP7A1) as compared to the liver bud before fusion. Further, the hepatic function of fused liver bud may be improved compared to that of conventional liver buds. For example, the fused liver bud can have higher gene expression levels of hepatic differentiation markers (e.g., ALB, OTC, CYP3A7 and GLUT2) while allowing for enhanced albumin production/transferrin production and ammonium metabolism as compared to conventional liver buds. Further, in the case where fused cell mass is transplanted into a living body, the graft survival rate may be improved compared to that of cell masses before fusion. Furthermore, in the case where fused cell mass has a vascular network, anastomosis and blood perfusion between the vascular network of the fused cell mass transplanted into a living body and the host vessels may be observed.

When the fused cell mass is transplanted into a human or a non-human animal, vascular networks can be formed in the transplanted fused cell mass, for example, tissue or organ and blood perfusion starts. As a result, a tissue or an organ with a highly ordered tissue structure can be generated. Therefore, it is possible to generate a tissue or an organ by transplanting the fused cell mass into a non-human animal. Regeneration or functional recovery of a tissue or an organ is also possible by transplanting the fused cell mass of the present invention into a human or a non-human animal. Examples of animals to be transplanted with the fused cell mass include human and non-human animals that are used, for example, as experimental animals, pet animals, working animals, race horses or fighting dogs; more specifically, mouse, rat, rabbit, pig, dog, monkey, cattle, horse, sheep, chicken, shark, devilfish, ratfish, salmon, shrimp, crab and the like may be enumerated. Moreover, in order to avoid immunorejection, the non-human animal as the recipient is preferably an immunodeficient animal. The site of transplantation of the fused cell mass may be any site as long as transplantation is possible. Specific examples of the transplantation site include, but are not limited to, the blood vessel, the intracranial space, the mesentery, the liver, the spleen, the kidney, the kidney subcapsular space, and the supraportal space. By transplanting the fused cell mass to a living body (human or non-human animal, etc.), it is possible to regenerate tissues or organs whose function has been lost or deteriorated. Further, if human tissues or organs are prepared in non-human animals by such transplantation, it is possible to use the human tissues or organs in drug discovery screening.

Alternatively, the fused cell masse may be cultured *in vitro* to be further improved in function and the resultant product may be used as an organ analogue or an organ in human biology, regenerative medicine or drug discovery screening.

In the present invention, it is possible to prepare vascularized organoids (liver buds) of large size by fusing cell masses. By an increase in size, the function of cell mass is improved and a vascular system resembling blood vessels in a living body (microvessels, arterioles/venules, etc.) can be formed. In the case where the cell mass is a liver bud, a bile duct structure may also be formed. In an example described later, a vascular network structure and/or a bile duct structure was formed by fusing liver buds *in vitro* as they had been formed from hepatocytes, mesenchymal cells and vascular cells. Therefore, the present invention provides an artificial organ that is prepared by fusing liver buds *in vitro* as they have been formed from hepatocytes, mesenchymal cells and vascular cells and which has a vascular network structure and/or a bile duct structure. In the artificial organ of the present invention, at least one of the hepatocyte, the mesenchymal cell and the vascular cell may be generated by directed differentiation from induced pluripotent stem cells.

It is possible to prepare a non-human chimeric animal by transplanting into a non-human animal a fused cell mass and engrafting the fused cell mass. The non-human animal (e.g., mouse) transplanted with the fused cell mass may mimic the physiological function of the biological species (e.g., human) from which the cell mass is derived.

Still further, the present invention provides a method of evaluating a drug, comprising using at least one member selected from the group consisting of a fused cell masse, a tissue or an organ prepared from a fused cell mass, and a non-human chimeric animal transplanted with a fused cell mass. Specific examples of drug evaluation include, but are not limited to, evaluation of drug metabolism (e.g., prediction of drug metabolism profiles), evaluation of drug efficacy (e.g., screening for drugs that are effective as pharmaceuticals), toxicity evaluation, and evaluation of drug interactions.

Evaluation of drug metabolism may be performed as follows. Briefly, a fused cell mass obtained by fusing cell masses prepared from human- or non-human animal-derived cells, a tissue or an organ prepared from the fused cell mass, or a non-human chimeric animal transplanted with the fused cell mass is individually administered with a candidate compound for pharmaceuticals and the resulting biological sample is then collected and analyzed, whereby a human drug metabolism profile can be obtained. As a result, prediction of the distribution/metabolism/excretion processes of pharmaceuticals in humans-this has been extremely difficult to achieve by conventional methods-becomes possible and it is expected that the development of safe and efficacious pharmaceuticals can be greatly accelerated.

Screening for drugs that are effective as pharmaceuticals may be performed as follows. Briefly, a fused cell mass obtained by fusing cell masses prepared from human- or non-human animal-derived cells, a tissue or an organ prepared from the fused cell mass or a non-human chimeric animal transplanted with the fused cell mass is administered with a novel candidate compound for pharmaceuticals. This enables subsequent analysis. As a result, a potential is expected for achieving great improvement in the precision of drug efficacy prediction for the case of actual administration to humans, which has been unsatisfactory in conventional *in vitro* tests.

Evaluation of toxicity may be performed as follows. Briefly, a fused cell mass obtained by fusing cell masses prepared from human- or non-human animal-derived cells, a tissue or an organ prepared from the fused cell mass or a non-human chimeric animal transplanted with the fused cell mass is administered with a test substance and, thereafter, histological damage markers or the like are measured. This makes it possible to improve the precision of damage prediction.

Evaluation of drug interactions may be performed as follows. Briefly, a fused cell mass obtained by fusing cell masses prepared from human- or non-human animal-derived cells, a tissue or an organ prepared from the fused cell mass or a non-human chimeric animal transplanted with the fused cell mass is administered with multiple drugs; then, each drug is examined for its pharmacokinetics such as distribution/metabolism/excretion processes, evaluated for its toxicity, and evaluated for its efficacy.

The fused cell mass prepared by the method of the present invention may be used as an active ingredient of a composition for regenerative medicine.

This composition for regenerative medicine may be transplanted *in vivo* into human or non-human animal to prepare a tissue or an organ. Regeneration or functional recovery of a tissue or an organ is also possible by transplanting the composition into a living body.

Upon transplantation of the composition for regenerative medicine into human or a non-human animal, the fused cell mass may differentiate into a tissue or an organ with vascular networks. In such vascular networks, blood perfusion may occur. It is believed that the occurrence of blood perfusion in vascular networks enables generation of a tissue or an organ with a highly ordered tissue structure equivalent or close to the tissue structure of adult tissues.

The composition for regenerative medicine may comprise a tissue vascularization promoter such as FGF2, HGF or VEGF, a gelatin sponge for hemostasis to cope with the bleeding from transplantation (product name: Spongel; Astellas Pharma), and a tissue adhesive for fixing transplanted tissues such as Bolheal (Teijin Pharma), Beriplast™ (CSL Behring), TachoComb™ (CSL Behring), collagen or Matrigel™.

### EXAMPLE

Hereinbelow, the present invention will be described in more detail with reference to the following Example.

### [Example 1]

### Experimental Methods

### - Culture of Human Induced Pluripotent Stem Cells (iPSCs)

Cell culture dishes or plates were coated with iMatrix-511 (Nippi, 0.7-0.9 µg/cm²) at 37°C for 1 hour and washed with PBS. Cryopreserved human iPSCs (TkDA strain and 1231 A3 strain, kindly provided by the University of Tokyo and Kyoto University, respectively) were soaked in warm water of 37°C for 2 minutes and thawed under shaking with hands. Cell stock solution was suspended in 9 volumes of StemFit medium (Ajinomoto) and centrifuged at 150-200 x g for 5 minutes. After removal of supernatant, cells were suspended in AK02 medium supplemented with Y-27632 (10 µM), and human iPSCs were seeded at densities of 0.36-1.8 x 10³ cells/cm². The medium was changed with AK02 medium at day 1 of culture and, since then, medium change was carried out every other day. About passaging cells, human iPSCs cultured for 1 week in cell culture dishes of 10 cm in diameter were washed with PBS. Then, 2 ml of Accutase was added and cells were detached by treatment at 37°C for 5 to 10 minutes. After addition of 2 ml of AK02 medium, cells were transferred into 15 ml tubes and centrifuged at 150-200 x g for 5 minutes. Supernatant was removed and the resultant cells were suspended in AK02 medium supplemented with Y-27632 (10 µM), and human iPSCs were seeded at densities of 0.36-1.8 x 10³ cells/cm².

### - Directed Differentiation from iPSC to Hepatic Progenitor Cell

Cell culture dishes or plates (Falcon) were coated with iMatrix-511 (Nippi, 0.4-0.6 µg/cm²) at 37°C for 1 hour and washed with PBS. Human iPSCs cultured for 1 week in cell culture dishes of 10 cm in diameter were washed with PBS. Then, 2 ml of Accutase was added and cells were detached by treatment at 37°C for 5 to 10 minutes. After cell harvesting and centrifugation, supernatant was removed, and the resultant cells were suspended in RPMI medium supplemented with penicillin/streptomycin (1%), B27 (2%), Wnt3a (50 ng/ml), Activin A (100 ng/ml) and Y-27632 (10 µM) and seeded in laminin-coated dishes at a density of 5-10 x 10⁴ cells/cm². At days 1 and 3 of culture, the medium was changed with RPMI medium supplemented with penicillin/streptomycin (1%), B27 (2%), Wnt3a (50 ng/ml), Activin A (100 ng/ml) and Sodium Butyrate (0.5 mM). At day 4 of culture, the medium was changed with RPMI medium supplemented with penicillin/streptomycin (1%), B27 (2%), Wnt3a (50 ng/ml) and Activin A (100 ng/ml). The cells at day 6 of culture were designated as endodermal cell Definitive Endoderm (DE). At days 6 and 8 of culture, the medium was changed with RPMI medium supplemented with penicillin/streptomycin (1%), B27 (2%), basic FGF (10 ng/ml) and BMP-4 (20 ng/ml). The cells at day 10 of culture were designated as hepatic progenitor cell Hepatic Endoderm (HE).

### - Directed Differentiation from iPSC to Vascular Endothelial Cell

Directed differentiation from iPSC to vascular endothelial cell was performed as described previously in the literature (Takebe et al., Cell Reports, 2017). Briefly, iPSCs were seeded in the same manner as described for the directed differentiation to hepatic progenitor cell, and cultured in StemFit medium supplemented with Y-27632 (10 µm) for one day. Next day, the medium was changed to DMEM/F12 medium supplemented with 1% Glutamax, 1% B27, CHIR99021 (8 µM) and BMP-4 (25 ng/ml). After a three-day culture, the medium was changed to StemPro-34 SFM medium supplemented with VEGF (200 ng/ml) and forskolin (2 µM). At day 7 of directed differentiation, the quality of the resultant cells was checked by confirming the expression of markers CD31 and CD144. Using StemPro-34 SFM medium supplemented with VEGF (50 ng/ml), the obtained vascular endothelial cells were passaged on fibronectin-coated culture dishes for expansion of culture.

### - Directed Differentiation from iPSC to Mesenchymal Cell

Directed differentiation from iPSC to mesenchymal cell was performed as described previously in the literature (Takebe et al., Cell Reports, 2017). Briefly, iPSCs were seeded at a density of 2-8 x 10^³ cells/cm² in the same manner as described for the directed differentiation to hepatic progenitor cell, and cultured in StemFit medium supplemented with Y-27632 (10 µm) for 4 to 6 days. Then, the medium was changed to DMEM/F12 medium supplemented with 1% Glutamax, 1% B27, CHIR99021 (8 µM) and BMP-4 (25 ng/ml). After a three-day culture, the medium was changed to DMEM/F12 medium supplemented with 1% Glutamax, 1% B27, Activin A (2 ng/ml) and PDGFBB (10 ng/ml). After three days, the medium was changed to StemPro-34 SFM medium supplemented with FGF2 (10 ng/ml) and PDGFBB (10 ng/ml), and the cells were cultured for three days.

### - Culture of Human Mesenchymal Stem Cells (MSCs)

Mesenchymal stem cells (hereinafter, abbreviated to MSCs; Lonza; 3-5 x 10^⁵ cells) suspended in MSCGM medium (Lonza) were seeded in a cell culture dish (10 cm in diameter). Medium change was carried out every three days. Seven days later, cells were washed with PBS and then 2 ml of Trypsin/EDTA (Gibco) was added. Cells were detached by treatment at 37°C for 5 minutes. MSCGM medium (2 ml) was added and cells were transferred into 15 ml tubes, followed by centrifugation at 150-200 x g for 5 minutes. After removal of supernatant, cells were suspended in HCM/EGM mixed medium and supplied for preparation of liver buds; the HCM/EGM mixed medium was such that HCM (EGF free) medium (Lonza) supplemented with FBS Gold (MP Biomedicals) 5%, HGF 10 ng/ml, Oncostatin M (R&D) 20 ng/ml and Dexamethazon 100 nM was mixed with EGM medium at 1:1.

### - Culture of Human Umbilical Vein Endothelial Cells (HUVECs)

Kusabira Orange-labeled human umbilical vein endothelial cells (hereinafter, abbreviated to HUVECs; Lonza; 3-5 x 10^⁵ cells) suspended in EGM medium (Lonza) or EGM-2 medium (Lonza) were seeded in a cell culture dish (10 cm in diameter). Medium change was carried out every three days. Seven days later, cells were washed with PBS and then 2 ml of Trypsin/EDTA (Gibco) was added. Cells were detached by treatment at 37°C for 5 minutes t. EGM medium or EGM-2 medium (2 ml) was added and cells were transferred into 15 ml tubes, followed by centrifugation at 150-200 x g for 5 minutes. After removal of supernatant, cells were suspended in the above-described HCM/EGM mixed medium and supplied for preparation of liver buds.

### - Preparation of Liver Buds

For preparation of small size liver buds, human iPSC-derived DE or HE (2.3-5 x 10⁵ cells), HUVEC or iPSC-EC (0.5-3.5 x 10⁵ cells), and MSC or iPSC-MC (0.5-3.5 x 10⁵ cells) were suspended in the HCM/EGM mixed medium and seeded in each well of a micro-patterned 24-well plate Elplasia RB 500 400 NA (Kuraray). With respect to conventional methods, human iPSC-derived DE or HE (5 x 10⁵ cells), HUVEC or iPSC-EC (3.5 x 10⁵ cells), and MSC or iPSC-MC (0.5 x 10⁵ cells) were suspended in the HCM/EGM mixed medium in each well of a 48-well plate and seeded on 50% Matrigel™ pre-solidified at 37°C (Matrigel: EGM=1:1, 150 ul/well in the 48-well plate). Liver buds were cultured at 37°C under 5% CO₂ for 24 hrs and used in liver bud fusion.

### - Liver Bud Fusion

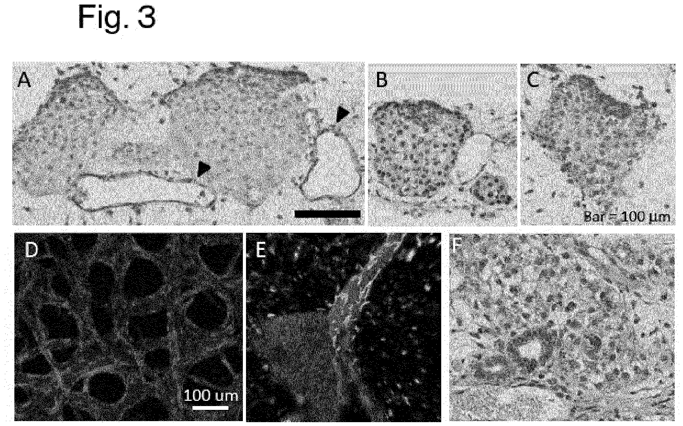

Twenty-four hours after preparation of liver buds, small size live buds were collected from micro-patterned well plate Elplasia RB 500 400 NA (Kuraray). The collected liver buds were located in proximity to each other on 24-well culture insert (Falcon), 12-well culture insert (Falcon) or 6-well culture insert (Falcon); or on PrimeSurface™ plate 96U, PrimeSurface™ plate 96V (Sumitomo Bakelite), ultra-low-attachment 96-well flat bottom plate with lid or 96-well transparent cell culture surface treated polystyrene microplate (Corning), or artificial blood vessel C-Porous™ porous tube made of PTFE (Chukoh Chemical Industries). When 24-well cell culture insert (Falcon) was used, each well contained 500 ul of the HCM/EGM mixed medium, with 600 small size liver buds or 2 conventional liver buds being located in each well. When 12-well cell culture insert (Falcon) was used, each well contained 1 ml of the HCM/EGM mixed medium, with 1200 small size liver buds being located in each well. When 6-well cell culture insert (Falcon) was used, each well contained 2 ml of the HCM/EGM mixed medium, with 2400 small size liver buds being located in each well. It is also possible to use a silicone gasket on the cell culture insert (Culture-Insert 2well/3well, ibidi). A silicone gasket was used in Figures 3, 9 and 10. The surface of each insert was coated with collagen I. The surface of the artificial blood vessel was coated with 0.1% gelatin. When PrimeSurface™ plate 96U, rimeSurface™ plate 96V, ultra-low-attachment 96-well flat bottom plate with lid, or 96-well transparent cell culture surface treated polystyrene microplate (Corning) was used, each well contained 200 ul of the HCM/EGM mixed medium, with 600 small size liver buds or 2 conventional liver buds being located in each well. The density of small size liver buds was 30000 spheroids/ml in each case. Subsequently, liver buds were cultured at 37°C under 5% CO₂ for 7 days. Immediately after location, as well as 1, 3, 7 and 9 days after location, photos were taken with a fluorescent microscope BZ-X710 (Keyence) and a confocal microscope SP5 or SP8. At day 9 of culture, liver buds were fixed with 4% PFA/PBS. After paraffin embedding, 7-10 µm thick sections were prepared and subjected to tissue analysis.

### - Transplantation of Liver Buds into Cranial Window

Immunodeficient NOD/SCID mice were purchased from Sankyo Lab. Co. After ketamine/xylazine mixed anesthetic or medetomidine hydrochloride/ midazolam/ butorphanol mixed solution was administered to the mice intraperitoneally, the skull was thinly cut with a dental microdrill along a circular path and the resultant circular portion was removed. After removal of the dura, physiological saline was added. Then, a custom-made circular slide glass (Matsunami) was mounted on the skull and sealed tightly with a dental material (Coe Tray Plastic). One week after preparation of this cranial window, the slide glass was removed, and small size liver buds or fused liver buds were transplanted on the surface of the brain and sealed. After transplantation, photos were taken chronologically with a fluorescence stereomicroscope (Carl Zeiss) or a confocal microscope SP5 (Leica) or SP8 (Leica). When it was necessary to observe the blood flow, Angiosense 680 (Perkin Elmer) was injected into the tail vein. Three weeks after transplantation, the transplant was fixed with 4% PFA/PBS. After paraffin embedding, 7-10 µm thick sections were prepared and subjected to tissue analysis.

### - Functional Evaluation of Fused Liver Bud with Quantitative RT-PCR

Small size liver buds prepared on Elplasia RB 500 400 NA (Kuraray) and fused liver buds were collected at day 10 of culture, and RNA was prepared and purified with PureLink RNA mini kit (Thermo Fisher Scientific). Then, cDNA was synthesized with iScript cDNA Synthesis Kit (Bio-Rad), followed by amplification and detection of genes using KAPA SYBR™ Fast qPCR kit (Nippon Genetics) and LightCycler™ 480 (Roche Life Science).

### - Functional Evaluation of Fused Liver Bud with ELISA

At day 10 of culture of fused liver buds, the medium was changed to a fresh medium. Twenty-four hours later, the medium was collected, and the levels of albumin and transferrin in culture supernatant were measured with commercial ELISA kits (from Bethyl Laboratories and Abcam, respectively).

### - Functional Evaluation of Urea/Ammonium Metabolic Pathways

At day 10 of culture of fused liver buds, the medium was changed to a fresh medium. Twenty-four hours later, the medium was collected, and the level of urea in culture supernatant was measured with QuantiChrom Urea Assay Kit. As regards ammonium metabolism, the collected medium was changed to 2 mM ammonium chloride-containing medium, which was collected after 24 hours. The level of ammonium in culture supernatant was measured with Amicheck (Arkray). The difference from the ammonium level in the medium immediately after addition of 2 mM ammonium chloride-containing medium was taken as the amount of ammonium metabolism.

### - Preparation of Paraffin Sections

The collected tissue was fixed in 4% paraformaldehyde/phosphate buffer (PBS) for 16 hours. After washing 3 times with PBS, the fixed tissue was dehydrated by immersing in 70% ethanol, 95% ethanol and 100% ethanol (twice), each for at least 30 minutes. Subsequently, the tissue was immersed in 50% ethanol/xylene solution, 100% xylene solution and 100% paraffin solution (twice), each for at least 30 minutes, under shaking at 65°C and then embedded in paraffin. The embedded tissue sample was sliced into 7-10 µm thick sections with a microtome. After slicing, the sample was dried at 37°C.

### - Hematoxylin-Eosin Stain

Dried paraffin sections were deparaffinized with xylene and gradually hydrophilized with 100% ethanol, 95% ethanol, 70% ethanol and Milli-Q™ water. After washing with running water, samples were stained in hematoxylin solution for 3 minutes. After washing with running water, samples were stained in eosin solution for 2 minutes. After washing with running water, samples were dehydrated with alcohol and cleared with xylene. Subsequently, a water-insoluble mounting medium was placed on the sample, which was covered with a cover glass for inclusion.

### - Immunohistochemical Analysis

Dried paraffin sections were deparaffinized with xylene and gradually hydrophilized with 100% ethanol, 95% ethanol, 70% ethanol and Milli-Q™ water. After antigen retrieval treatment in 10 mM citrate buffer (pH 6.0) at 121°C for 15 minutes, samples were treated in 3% hydrogen peroxide water/methanol solution for 10 minutes to inactivate endogenous peroxidase. After blocking with Blocking Ones solution (Nacalai Tesque) at room temperature for 1 hour, samples were reacted with diluted primary antibody (hCD31 antibody (M0823, DAKO), hAlbmin antibody (A6684, Sigma), HNF4α antibody (sc-6556, Santa Cruz Biotechnology) or Vimentin antibody (M7020, DAKO)) at room temperature for 1 hour or at 4°C for overnight. After washing 3 times with PBS, samples were reacted with HRP-conjugated or fluorescence-labeled secondary antibody at room temperature for more than 30 minutes. After reaction with the fluorescence-labeled secondary antibody, samples were washed 3 times with PBS, subjected to nuclear staining with DAPI and then included for microscopic observation. After reaction with the HRP-conjugated secondary antibody, samples were reacted with DAB reagent (DAKO) for 2 to 10 minutes to conduct color development. After washing with purified water, samples were stained with hematoxylin for 3 minutes. After washing with running water for 10 minutes, samples were reacted with 0.1% Sodium Bicarbonate for 5 minutes, dehydrated with alcohol and cleared with xylene. Subsequently, a water-insoluble mounting medium was placed on the sample, which was covered with a cover glass for inclusion.

### - Calculation of Area Occupied by Cell Masses as Relative to Seeded Plane

For calculation of the area occupied by cell masses, image analysis software ImageJ 1.51s (National Institutes of Health, U.S.A., downloadable from http://imagej.nih.gov/ij) was used. First, the entire seeded plane containing cell masses is selected. Then, after proceeding from Image→Adjust→Threshold, B&W is selected at Color selection to thereby change images to monochromes. Under this condition, cell masses become white and seeded plane black. When the minimum value of threshold (upper) is set at 130 and the maximum value of threshold (lower) at 255, the ratio (%) of seeded plane excluding the portion occupied by cell masses is displayed below the graph at the left of the threshold screen. Difference from 100% was calculated as the area occupied by cell masses (%).

### Experimental Results

### - Aggregation and Fusion of Human iPSC Liver Buds

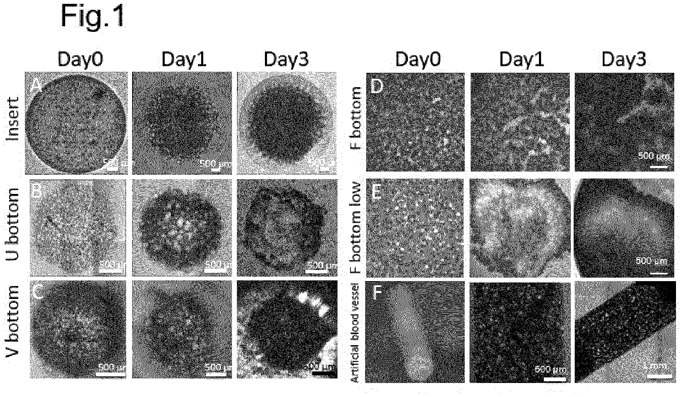

A plurality of small size liver buds (approx. 80-150 µm) prepared on Elplasia plate or conventional liver buds (approx. 1-2 mm) prepared on Matrigel™ were transferred to cell culture insert, U bottom plate and V bottom plate one day after the preparation. Then, aggregation and fusion of liver buds were observed chronologically up to day 7. As a result, starting from day 1 of fusion, aggregation and fusion of small size liver buds were observed on cell culture insert, U bottom plate, V bottom plate, F bottom plate, low attachment F bottom plate and artificial blood vessel (Figs. 1A-1F). Partial fusion of liver buds was observed on F bottom plate. The size of fused liver bud was about 4 mm on cell culture insert, about 2 mm on V bottom plate, about 3 mm on low attachment F bottom, and about 3 mm on artificial blood vessel. As regards the vascular network structure, it was retained by the fusion liver buds prepared on cell culture insert and artificial blood vessel for at least three days after fusion (Figs. 1A and 1F).

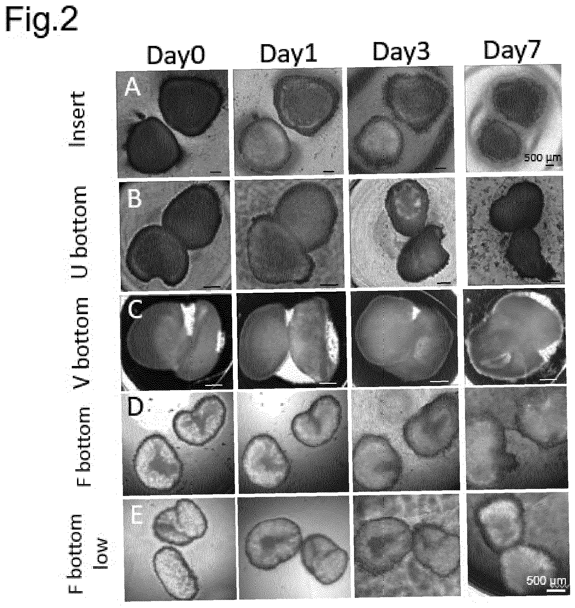

On the other hand, when conventional liver buds were used, the matrix surrounding the liver buds prevented them from aggregating and until day 7, no fusion was observed on U bottom plate, V bottom plate, cell culture insert, F bottom plate and low attachment F bottom plate (Figs. 2A-2E).

### - Reconstitution of Tissue in Fused Liver Bud

Upon tissue analysis, not only microvessels of 5-20 µm in diameter but also blood vessels of arteriole/venule level which were 100 µm or more in diameter were observed to have been reconstituted in fused liver buds (Fig. 3A). Also observed were a hepatocyte marker HNF4α and a human albumin-positive hepatocyte cluster (Figs. 3B and 3C). Reconstitution of blood vessels was also confirmed by imaging using a fluorescent protein Kusabira orange-labeled iPS-derived vascular endothelial cells (Figs. 3D and 3E). When images taken with a confocal microscope were subjected to 3D rendering by Imaris, it was confirmed that Vimentin-positive pericyte-like cells (yellow) are present around blood vessels (red) (Fig. 3E). In the case where fused liver buds had been transplanted into a cranial window, a bile duct structure was also observed in addition to hepatocytes (Fig. 3F).

### - Functional Analysis of Fused Human iPSC Liver Bud

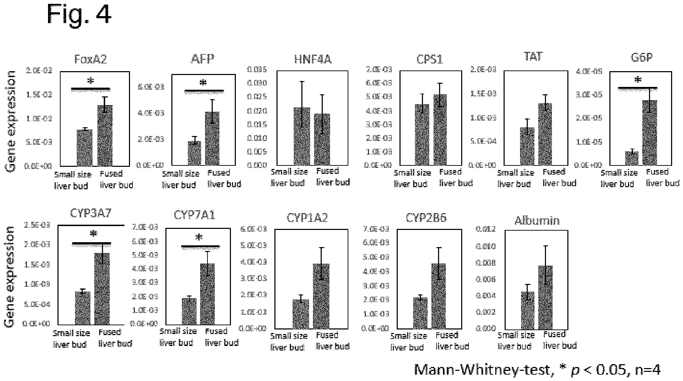

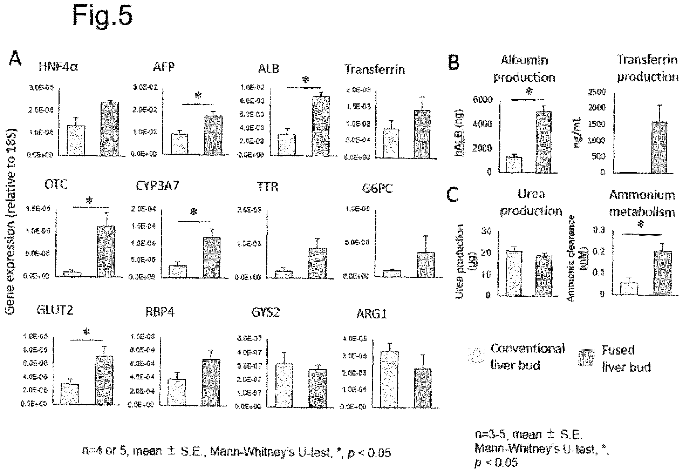

At day 8 of culture, RNA was collected both from small size liver buds and from fused liver buds. A total of 11 types of hepatic differentiation markers were subjected to gene expression analysis by qRT-PCR. The results revealed that the gene expression levels of hepatic differentiation markers FoxA2, AFP, CYP3A7 and CYP7A1 were significantly raised in fused liver buds (Mann-Whitney's U-test, p <0.05) (Fig. 4). Similar comparison with conventional liver buds was also conducted. The results revealed that the gene expression levels of hepatic differentiation markers ALB, AFP, OTC, CYP3A7 and GLUT2 were significantly increased in fused liver buds (Fig. 5A). Further, it was confirmed that albumin production capacity, transferrin production capacity and ammonium metabolism were increased in fused liver buds (Mann-Whitney's U-test, p <0.05) (Fig. 5B).

### - In Vivo Transplantation Analysis of Fused Human iPSC Liver Bud

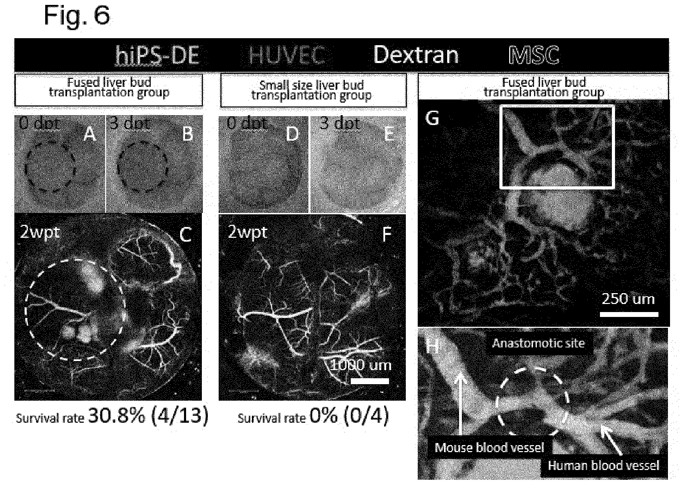

At day 4 of culture, small size liver buds and fused liver buds were respectively transplanted under a mouse cranial window (Figs. 6A-6F). By using this cranial window transplantation technique, the anastomosis between vascular endothelial cells within the transplant and the host's blood vessels, the growth of hepatic progenitor cells within the transplant and other events can be observed chronologically while the host animal is alive. Whereas the grafts in the fused liver bud transplantation group had a survival rate of 30.8% 2 weeks after transplantation, no engraftment was observed in the small size liver bud transplantation group (n=13 and n=4 for the respective groups) (Figs. 6A-6F). In the fused liver bud transplantation group, anastomosis and blood perfusion between the human blood vessel network formed within the fused liver bud and the host's blood vessels were observed, indicating that the human blood vessel network reconstituted within the fused liver bud was functioning (Figs. 6G-6H)

### - Examination of the Proportions of Three Constituent Cell Types in Fused Human iPSC Liver Bud

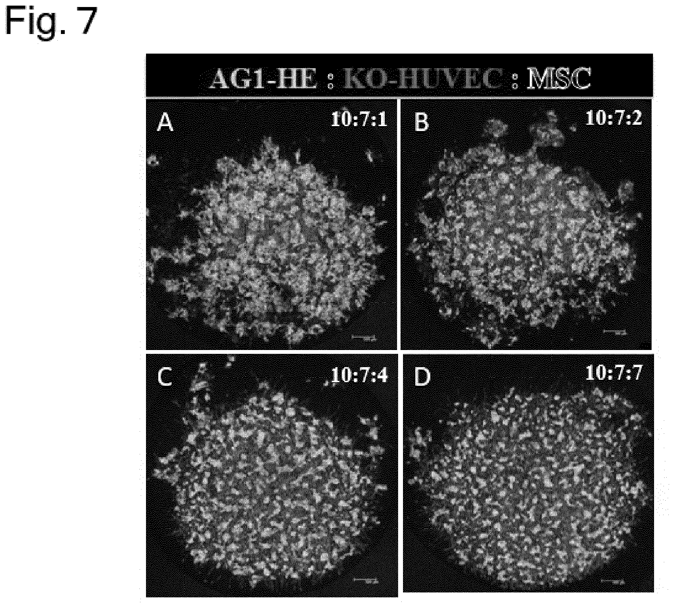

Fused liver buds were prepared using different proportions of three constituent cell types (iPS-HE : HUVEC : MSC = 10:7:1, 10:7:2, 10:7:4 and 10:7:7). As a result, a tendency was observed such that a higher MSC ratio gave blood vessels having a finer network structure (Figs. 7A-7D). This result revealed that the vascular network structure in fused liver buds can be controlled by changing the proportion of MSC.

### - Examination of Size Control of Fused Human iPSC Liver Bud

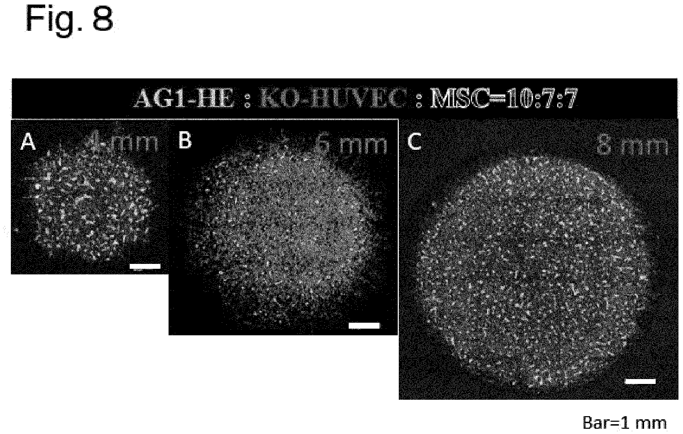

The present inventors examined the relationship between the number of small size liver buds to be fused and the size of the resultant fused liver bud. When 600 small size liver buds were fused, the diameter of the resultant fused liver bud was about 4 mm (Fig. 8A). When 1200 small size liver buds were fused, the diameter of the resultant fused liver bud was about 6 mm (Fig. 8B). When 2400 small size liver buds were fused, the diameter of the resultant fused liver bud was about 8 mm (Fig. 8C). These results revealed that the size of a fused liver bud can be controlled by the number of small size liver buds to be fused.

### - Examination of Cell Mass Seeding Density Used to Prepare Fused Human iPSC Liver Bud

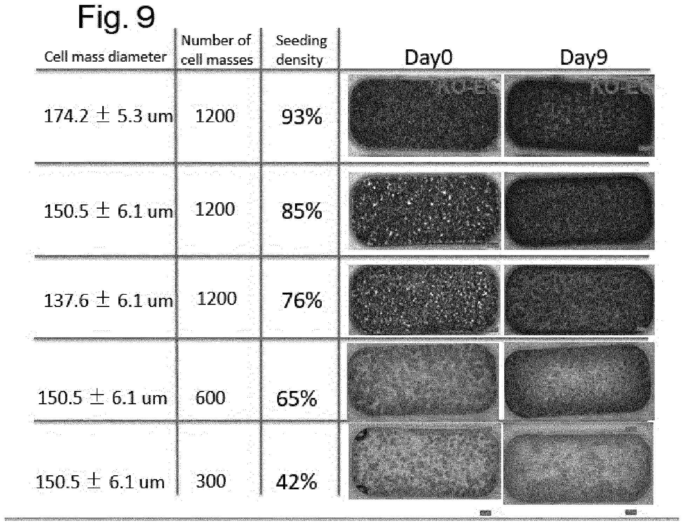

The present inventors examined the size of small size liver buds and their seeding density. When 1200 small size liver buds measuring 174 µm, 150 µm or 137 µm in diameter were seeded on a 21 mm³ plane capable of cell adhesion, the respective seeding densities were found to be 93%, 85% and 76% (Fig. 9). When 600 or 300 small size liver buds measuring 150 µm in diameter were seeded on a 21 mm³ plane capable of cell adhesion, the respective seeding densities were found to be 65% and 42% (Fig. 9). Further, at day 9 of culture, fused liver buds were obtained and vascular formation was observed in all groups (Fig. 9). When the seeding density was less than 40%, no uniform fusion of cell masses could be achieved.

### - Examination of Cell Proportions Used to Prepare Fused Liver Bud

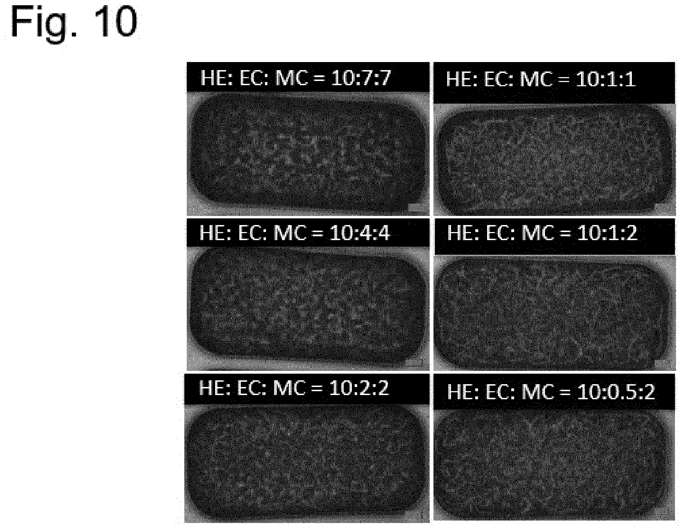

The present inventors examined the cell proportions of liver bud materials (HE, EC and MC). With the proportions of HE:EC:MC being varied as 10:7:7, 10:4:4, 10:2:2, 10:1:1, 10:1:2 and 10:0.5:2, cell masses were prepared and then located at high densities as described above (Fig. 10). At day 9 of culture, fused liver buds were obtained and vascular formation was confirmed in all groups (Fig. 10). A tendency was recognized such that as the proportion of EC or MC decreased, an increased number of reconstituted thin blood vessels was observed (Fig. 10). When the proportion of MC was unduly small (EC:MC≤1:0.01) or when the proportion of EC was unduly small (EC:MC≤0.01:1), cell masses were fused but no vascular network was found to form.

All publications, patents and patent applications cited herein are incorporated herein by reference in their entirety.

### INDUSTRIAL APPLICABILITY

The present invention is applicable to human biology, regenerative medicine, drug evaluation and so on.

## Claims

1. A method of fusing cell masses, comprising seeding cell masses on a plane capable of cell adhesion and culturing the cell masses as a culture medium is fed from both the obverse and reverse sides of the cell mass-seeded plane.

2. The method of claim 1, wherein cell masses are seeded on the plane capable of cell adhesion in such a manner that the ratio of the area occupied by the cell masses to the seeded plane is 40 to 100%.

3. The method of claim 1 or 2, wherein the cell mass comprises vascular cells and/or mesenchymal cells.

4. The method claim 3, wherein the ratio of mesenchymal cells to vascular cells is 1 : 0.01-100.

5. The method of claim 4, wherein the ratio of mesenchymal cells to vascular cells is 1 : 0.1-10.

6. The method of any one of claims 1 to 5, wherein the resultant fused cell mass is forming a vascular network structure.

7. The method of any one of claims 1 to 6, wherein the cell mass is an organ bud.

8. The method of claim 7, wherein the organ bud has been formed from tissue or organ cells, mesenchymal cells and vascular cells.

9. The method of claim 8, wherein the ratio of tissue or organ cells, mesenchymal cells and vascular cells is 10 : 0.1-10 : 0.1-10.

10. The method of claim 9, wherein the ratio of mesenchymal cells to vascular cells is 1 : 0.1-10.

11. The method of any one of claims 7 to 10, wherein the organ bud has been formed in a culture vessel having a non-cell-adhesive plane.

12. The method of any one of claims 7 to 11, wherein the organ bud is a liver bud and the fused liver bud is forming a vascular network structure.

13. The method of claim 12, wherein the vascular network structure comprises microvessels and/or arterioles/venules.

14. The method of any one of claims 6 to 13, wherein the organ bud is a liver bud and the fused liver bud is forming a bile duct structure.

15. A method of preparing a fused cell mass, comprising seeding cell masses on a plane capable of cell adhesion and culturing the cell masses as a culture medium is fed from both the obverse and reverse sides of the cell mass-seeded plane to thereby fuse the cell masses.

16. A method of constructing a vascular network structure, comprising seeding cell masses on a plane capable of cell adhesion and culturing the cell masses as a culture medium is fed from both the obverse and reverse sides of the cell mass-seeded plane to thereby form a vascular network structure.

17. An artificial organ that is prepared by fusing liver buds *in vitro* as they have been formed from hepatocytes, mesenchymal cells and vascular cells and which has a vascular network structure and/or a bile duct structure.

18. The artificial organ of claim 17, wherein at least one of the hepatocyte, the mesenchymal cell and the vascular cell has been generated by directed differentiation from induced pluripotent stem cells.
